Europäisches Patentamt

European Patent Office

Office européen des brevets

(11) Publication number: **0 289 186**
**A2**

---

## (12) EUROPEAN PATENT APPLICATION

(21) Application number: **88303462.1**

(51) Int. Cl.⁴: **A61K 37/43**

(22) Date of filing: **18.04.88**

---

(30) Priority: **23.04.87 US 41397**

(43) Date of publication of application:
**02.11.88 Bulletin 88/44**

(84) Designated Contracting States:
**AT BE CH DE ES FR GB GR IT LI LU NL SE**

(71) Applicant: **INTERNATIONAL MINERALS AND CHEMICAL CORPORATION**
**P.O. Box 207 1401 South Third Street**
**Terre Haute Indiana 47808(US)**

(72) Inventor: **Howard, David K.**
**3238 N. Daniels Court**
**Arlington Heights Illinois 6004(US)**
Inventor: **Maccecchini, Maria-Lusia, Dr.**
**2400 Chestnut Street**
**Philadelphia Pennsylvania 19103(US)**
Inventor: **Schricker, Brain R., Dr.**
**R.R. 25 7414 Troy Court**
**Terre Haute Indiana 47802(US)**

(74) Representative: **Holmes, Michael John et al**
**Frank B. Dehn & Co. Imperial House 15-19**
**Kingsway**
**London WC2B 6UZ(GB)**

---

(54) **Process for increasing the growth rate and enhancing the feed efficiency of meat producing livestock.**

(57) Growth rate of meat-producing livestock is increased and feed efficiency enhanced by periodic injection of a substance having growth hormone releasing factor (GRF) activity. The livestock are injected at least twice daily during their growth period and the injections are substantially uniformly spaced with respect to time. The total amount of the GRF substance administered per day is substantially equivalent in GRF activity to between about 1 and 100 micrograms human GRF per kilogram body weight of the livestock per day, preferably 30-50 micrograms human GRF per kilogram body weight of the livestock per day.

EP 0 289 186 A2

# PROCESS FOR INCREASING THE GROWTH RATE AND ENHANCING THE FEED EFFICIENCY OF MEAT-PRODUCING LIVESTOCK

## BACKGROUND OF THE INVENTION

### Field of the Invention

The present invention relates to a process for increasing the growth rate and enhancing the feed efficiency of meat-producing livestock.

### Description of the Background Art

For economic reasons, it is desirable to maximize the growth rate and enhance the feed efficiency of meat-producing livestock during commercial husbandry thereof.

Numerous attempts have been made to increase the growth rate of meat-producing livestock involving administration of various substances, including antibiotics (see, e.g., U.S. Patent No. 4,478,935), resorcylic acid lactone derivatives (see, e.g., U.S. Patent No. 4,004,978), and quinoxaline derivatives (see, e.g., U.S. Patent No. 4,012,512).

There also has been some interest in attempting to increase growth rates of livestock utilizing growth hormone releasing factors (GRF) and analogs thereof. Growth hormone releasing factors are neurohumoral substances that stimulate secretion of growth hormone in animals. For example, the following U.S. patents suggest the possibility of utilizing GRF and analogs thereof to promote growth in livestock: 4,517,181; 4,518,586; 4,528,190; 4,529,595; 4,562,175; 4,563,352; 4,585,756 and 4,595,676. Although the above patents suggest the possibility of promoting growth of livestock with GRF, as well as the possibility of administering GRF intravenously, subcutaneously and intramuscularly, there is no indication in the references that GRF could in fact promote growth and enhance feed efficiency if administered to livestock, and if so, what a suitable regimen for administering GRF to livestock would be to actually achieve growth promotion and feed efficiency enhancement.

Most in vivo studies concerning GRF have involved rats, but reported results have been far from clear cut. For example, in unanaesthetized rats, GRF inducement of growth hormone (GH) secretion has been found to be erratic, whereas in rats anaesthetized with pentobarbital and pretreated with anti-rat-GRF antibodies and antisomatostatin antibodies, growth hormone response was not desensitized by repetitive pulses of GRF. Coude et al., Trends in Biotechnology, 2(4):83-88 (1984). However, these researchers found that periodic (pulsatile) injection of GRF every two hours in man does desensitize the GH response. Comparably ambiguous and/or contradictory findings are reported in the following references: Clark et al., Nature, 314:281-283 (1985); McCormick et al., Endocrinology, 117(1):97-105 (1985); Clark et al., J. Encocrinology, 106:281-289 (1985); Wehrenberg et al., Annual Review of Pharmacology and Toxicology, 25:463-483 (1985); Hulse et al., Clinical Research, 33(1): 115A (1985); Hummelink et al., Pediatric Research, 18(11):1210 (1984); Borges et al., J. Clinical Endocrinology and Metabolism, 59(1):1-6 (1984); Gelato et al., J. Clinical Endocrinology and Metabolism, 61(3):444-450 (1985); Hizuka et al., Endocrinology Japan, 31(6):697-704 (1984).

In cattle, GRF has been studied with regard to its effects on growth hormone release and milk production (lactation), but the results have been inconclusive. Baile et al., Federation Proceedings, 44:1357 (1985); Enright et al., J. Dairy Science, 69:341-351 (1985); Lapierre et al., Federation Proceedings, 44:1358 (1985); Mosley et al., J. of Animal Science, 58(2):430-435 (1984).

In another study, Baile et al. further found that in sheep, multiple injections of GRF result in diminished growth hormone response. 1984 Federation Proceedings, 43(3) Abstract 2019 (1984).

Although the results of animal studies involving GRF have heretofore proven ambiguous and at times contradictory, the discovery of a process for increasing the growth rate and enhancing the feed efficiency of meat-producing livestock is highly desirable.

## SUMMARY OF THE INVENTION

In accordance with the present invention, a process for increasing the growth rate and enhancing the feed efficiency of meat-producing livestock comprises administering a substance having growth hormone releasing factor (GRF) activity to meat-producing livestock during a growth period of the livestock. The GRF substance is administered to the livestock by periodic injection of the substance with injections at least twice each day during the growth period, the injections being substantially uniformly spaced with respect to time. The total amount of the GRF substance administered per day is substantially equivalent in GRF activity to at least about 30 micrograms human GRF per kilogram body weight of the livestock per day.

## DESCRIPTION OF THE PREFERRED EMBODIMENTS

Despite ambiguous and contradictory reports in the past, Applicants have discovered that substances having growth hormone releasing factor (GRF) activity can be utilized to increase the growth rate and enhance feed efficiency of meat-producing livestock, such as cattle, swine, sheep and poultry.

Mammalian growth hormone releasing factors are peptides that are released by the pancreas and hypothalmus to stimulate secretion of growth hormone (GH). In accordance with the invention, substances having growth hormone releasing factor activity include natural GRFs derived by known extraction and subsequent concentration techniques from human, porcine, ovine or bovine tissue and the like, as well as synthetic GRFs and GRFs produced by recombinant DNA methods. Also encompassed within the invention are biologically active fragments of GRFs and GRF analogs, which are substantially functionally equivalent to GRFs, but with a chemical structure that varies in some way. Various substances having GRF activity are disclosed in U.S. Patent Nos. 4,517,181; 4,518,586; 4,528,190; 4,529,595; 4,562,175; 4,563,352; 4,585,756 and 4,595,676, incorporated herein by reference.

In accordance with the present invention, a substance having GRF activity is administered to meat-producing livestock by periodic injection of the GRF substance at least twice each day during a growth period of the livestock. The injections are substantially uniformly spaced with respect to time, such as, for example, injections twice each day every 12 hours, injections three times each day every 8 hours, injections four times each day every 6 hours, injections six times each day every four hours, and injections eight times each day every three hours. Applicants have found administration at 2, 4 and 8 times a day suitable in accordance with the invention, although economic considerations may dictate other schedules within the scope of the invention, such as, for example, 3 or 6 times a day.

When utilizing human GRF in accordance with the present invention, the total amount of the human GRF administered per day is between about 1 and 100 micrograms human GRF per kilogram body weight of the livestock, preferably about 30-50 micrograms human GRF per kilogram body weight of the livestock per day. Advantageously, each injection contains about the same dosage of the GRF substance with the daily dosage of GRF about evenly divided among injections. For example, in sheep, two injections per day of 20 micrograms GRF per kilogram body weight per injection, four injections per day of 10 micrograms GRF per kilogram body weight per injection or eight injections per day of 5 micrograms GRF per kilogram body weight per injection, are suitable.

The total amount of the GRF substance administered per day can exceed 40 micrograms GRF per kilogram body weight of the livestock. However, Applicants have found that greater than about 40 micrograms human GRF per kilogram body weight per day does not result in a substantially greater increase in growth rate and feed efficiency.

Although the invention has been specifically described in connection with utilizing human GRF to increase the growth rate and enhance the feed efficiency of sheep, it is to be understood that other GRF substances as defined above are encompassed within the process of this invention, and that the invention further encompasses increasing the growth rate and enhancing the feed efficiency of other livestock utilizing periodic administration of GRF substances. While other GRF substances may vary in potency from human GRF, an amount equivalent in potency to human GRF can easily be determined without undue experimentation utilizing routine dose level testing methods. Thus, in accordance with the invention, the total amount of GRF substance administered to livestock per day is substantially equivalent in GRF activity to between about 1 and 100 micrograms human GRF per kilogram body weight of the livestock per day, preferably at least about 30-50 micrograms human GRF per kilogram body weight of the livestock per day.

The invention is further illustrated by the following examples, which are not intended to be limiting.

EXAMPLE I

Studies were conducted to determine suitable dosage levels of injected GRF to increase growth hormone concentration in the blood of young wether lambs, and to determine the effects of varying the frequency of administration to ellicit growth hormone release over a 24-hour interval.

The results of these studies demonstrated that increasing levels of GRF administration result in increasing concentrations of serum growth hormone at four equally spaced injections per day of up to a maximum of 10 μg human GRF per kg body weight per injection, whereas there were no differences in growth hormone serum levels of animals treated with 10 or 20 μg human GRF per kg body weight per injection four times per day. According to these studies, serum growth hormone levels over 24 hours were maximized at four injections of 10 μg per kg every 6 hours, or eight injections of 5 μg per kg every 3 hours.

EXAMPLE II

Another sheep study was conducted to determine the effect of exogeneous administration of human GRF or ovine growth hormone on weight gain, feed utilization and composition of gain. Sheep were injected twice daily with saline as negative control, twice daily with ovine growth hormone at 100 μg per kg body weight per injection as positive control, twice daily with human GRF at 20 μg per kg body weight per injection and four times daily with human GRF at 10 μg per kg body weight per injection. After 5 weeks, weight gain and feed efficiency data indicated that the GRF-treated sheep gained weight at a rate 20% better than the saline negative control and 10% better than the ovine growth hormone positive control. The study also indicated that feed efficiency was also improved over positive control, 4% for the sheep injected twice daily and 16% for the sheep injected four times daily.

EXAMPLE III

Studies were conducted to determine the dosage level of injected GRF below which substantial increases in growth hormone in the blood of wether lambs did not occur. The results are shown in Table 1 below.

TABLE 1

| μg GRF/Kg Body Weight | Growth Hormone Release[1] | Fold Increase |
|---|---|---|
| 0 | 8.9 | 0 |
| 1 | 28.9 | 3.3 |
| 5 | 91.5 | 10.3 |
| 10 | 138.0 | 15.5 |

1  Cumulative growth hormone release for 150 minutes after administration of GRF

The results of these studies demonstrate that a dose of as little as 1 μg human GRF per Kg body weight resulted in an approximately 3-fold increase in the concentration of growth hormone in the blood. Growth hormone concentrations in the blood increased linearly with increasing doses of human GRF up to a dose of 10 μg human GRF per Kg body weight, which dose resulted in an approximately 16-fold increase in the concentration of growth hormone in the blood.

EXAMPLE IV

Studies were conducted to determine the effect of administration of 50 μg human GRF per Kg body weight on the concentration of growth hormone in the blood of wether lambs, see Table 2 below.

TABLE 2

| Time after Administration of 50 μg/Kg Body Weight GRF | Growth Hormone Concentration (ng/ml) | | | Fold Increase |
|---|---|---|---|---|
| | Animal #1 | Animal #2 | Mean | |
| 0 minutes | 1.3 | 2.4 | 1.9 | 0 |
| 10 | 78.8 | 18.3 | 48.6 | 25.6 |
| 20 | 46.0 | 14.6 | 30.3 | 15.9 |
| 30 | 19.3 | 8.6 | 13.5 | 7.1 |
| 40 | 4.8 | 8.1 | 6.5 | 3.4 |
| 50 | 4.8 | 5.7 | 5.3 | 2.8 |
| 60 | 3.0 | 6.4 | 4.7 | 2.5 |

The results shown in Table 2 indicate that administration of 50 μg human GRF resulted in an approximately 26-fold increase in the concentration of growth hormone in the blood.

**Claims**

1. A process for increasing the growth rate and/or enhancing the feed efficiency of meat-producing livestock, comprising administering a substance having growth hormone releasing factor (GRF) activity to meat-producing livestock during a growth period of the livestock, the substance being administered to the livestock by periodic injection of the substance with injections at least twice each day during the growth period, the injections being substantially uniformly spaced with respect to time, wherein the total amount of said substance administered per day is substantially equivalent in GRF activity to between about 1 to 100ug human GRF per kg body weight of the livestock per day.

2. The process of claim 1 wherein said total amount has a GRF activity substantially equal to at least about 30-50ug human GRF per kg body weight of the livestock per day.

3. The process of claim 1 or claim 2 wherein each injection contains about the same dosage of said substance.

4. The process of any one of claims 1 to 3 wherein said livestock is cattle, swine, sheep or poultry.

5. The process of any one of claims 1 to 4 wherein the substance is injected two times per day.

6. The process of any one of claims 1 to 4 wherein the substance is injected three times per day.

7. The process of any one of claims 1 to 4 wherein the substance is injected four times per day.

8. The process of any one of claims 1 to 4 wherein the substance is injected six times per day.

9. The process of any one of claims 1 to 4 wherein the substance is injected eight times per day.

10. The process of any one of claims 1 to 9 wherein the substance is natural or recombinant human GRF, bovine GRF, porcine GRF or ovine GRF, or a biologically active fragment or analog thereof.

11. The method of any one of claims 1 to 9 wherein the substance is natural or recombinant human GRF, or a biologically active fragment or analog thereof, and the livestock is sheep.

12. Use of a substance having growth hormone releasing factor (GRF) activity for preparation of a composition for increasing the growth rate and/or enhancing the feed efficiency of meat producing livestock by a process according to claim 1.